Europäisches Patentamt

European Patent Office

Office européen des brevets

(11) Veröffentlichungsnummer: **0 195 351**
**A2**

## (12) EUROPÄISCHE PATENTANMELDUNG

(21) Anmeldenummer: **86103236.5**

(22) Anmeldetag: **11.03.86**

(51) Int. Cl.⁴: **A 61 K 6/04**

(30) Priorität: **22.03.85 DE 3510331**

(43) Veröffentlichungstag der Anmeldung:
**24.09.86 Patentblatt 86/39**

(84) Benannte Vertragsstaaten:
**AT FR GB IT**

(71) Anmelder: **Thyssen Edelstahlwerke AG**
**Thyssenstrasse 1**
**D-4000 Düsseldorf(DE)**

(72) Erfinder: **Rademacher, Leo, Dr.-Ing.**
**Hasenkampweg 29**
**D-5810 Witten(DE)**

(54) Dental-Gusslegierung.

(57) Die Erfindung betrifft eine Kobalt-Chrom-Gußlegierung, also eine Nicht-Edel-Metallegierung (NEM-Legierung), aus der Gerüste und Platten für herausnehmbare Dentalprothesen gegossen werden. Die Legierungszusammensetzung ist (in Massen-%):

0,1 bis 1,0 % Kohlenstoff
0,05 bis 0,5 % Stickstoff
0,5 bis 3,0 % Silizium
0,3 bis 10,0 % Mangan
20 bis 35 % Chrom
2 bis 10 % Molybdän
5 bis 40 % Eisen

Rest mindestens jedoch 20 % Kobalt einschließlich unvermeidbarer Verunreinigungen.

0195351

## Kobalt-Chrom-Gußlegierung für Dentalzwecke

Nicht-Edel-Metall-(NEM)-Gußlegierungen auf Kobalt-Chrom-Basis wurden für Dentalzwecke schon vor mehr als 50 Jahren eingeführt. Sie enthielten neben Kobalt und Chrom als wesentliche Legierungsbestandteile noch Molybdän und vereinzelt auch Nickel im teilweisen Austausch gegen Kobalt (siehe: F.R. Morral: Journal of Materials 1 (1966), Seite 384 bis 412).

Inzwischen haben sich Werkstoffe auf einer Legierungsbasis mit rd. 62 % Co, 30 % Cr und 5 % Mo durchgesetzt. Sie haben die ursprünglich verwendeten Edelmetall-Legierungen praktisch völlig verdrängt. Diesen Vorzug haben die Co-Cr-Legierungen ihren sowohl in klinischer als auch in technologischer Hinsicht hervorragenden Eigenschaften für die gekennzeichnete Indikation zu verdanken. Ein nicht unwesentlicher Vorteil der NEM-Legierungen gegenüber den Edelmetall-Legierungen besteht auch in ihrem um Größenordnungen geringeren Preis.

Die Materialkosten sind inzwischen durch die aus volkswirtschaftlicher Sicht immer notwendigere und deshalb von allen verantwortlichen Stellen immer eindringlicher geforderte Kostendämpfung im Gesundheitswesen in den Vordergrund des Interesses gerückt worden. Für die Werkstoffentwicklung hat dadurch die Senkung der Kosten einen sehr hohen Stellenwert erhalten.

Neben diesen rein wirtschaftlichen Gesichtspunkten waren die Werkstoffeigenschaften nicht zu vernachlässigen, die zur Eignung einer Metallegierung für die erwähnte Indikation eingehalten werden müssen.

-2-

Für die Anforderungen an die Werkstoffeigenschaften sind die Empfehlungen der International Organization for Standardization (ISO) als richtungsweisend anzusehen, weil die erwähnten NEM-Gußlegierungen international vermarktet werden. Die betreffende Norm ISO 6871 "Dental base metal casting alloys" befindet sich zur Zeit im Druck. Der hierfür vorliegende Entwurf ISO/DIN 6871 vom 12.07.1984 enthält folgende bereits als endgültig geltende und für die erfindungsgemäß zu verwendende Legierung als maßgeblich anzusehenden Empfehlungen:

- die Anforderungen an die chemische Zusammensetzung beinhalten neben Legierungen mit einem Gesamtgehalt an Chrom, Kobalt und Nickel von mindestens 85 % alternativ auch Legierungen mit einer anderen Zusammensetzung, vorausgesetzt, daß sie eine befriedigende Übereinstimmung mit den Anforderungen an die Toxizität und an die Korrosionsbeständigkeit aufweisen. Zu diesen Anforderungen wird im weiteren angegeben, daß die Legierungen weder Bestandteile mit schädlicher Wirkung für den Prothesenträger abgeben, noch sichtbare Zeichen von Korrosion aufweisen dürfen, nachdem sie ein Jahr lang einer oralen Umgebung ausgesetzt waren. Darüber hinaus wird noch für berylliumhaltige Legierungen empfohlen, daß der Gehalt 2 % Be nicht überschreiten soll;

- für die mechanischen Eigenschaften beschränkt sich die Norm auf einen Mindest-02 %-Dehngrenze von 500 N/mm$^2$ und auf einen Mindest-Bruchdehnungswert $A_5$ = 3 %;

- die Vergießbarkeit soll gewährleistet sein.

Der Preis der gebräuchlichen Kobalt-Chrom-Gußlegierungen wird im wesentlichen durch den Gehalt an Kobalt bestimmt. Dies ist nicht nur auf seinen weit überwiegenden Anteil zurückzuführen, sondern auch auf seinen im Vergleich zu den übrigen Elementen hohen Preis. Hinzukommt, daß in den zurückliegenden Jahren sehr erhebliche spekulative, wenn auch vorübergehende Steigerungen des Kobalt-Weltmarktpreises die Werkstoffkosten der Kobalt-Chrom-Legierungen zeitweise sehr stark belastet haben.

Aus diesem Grunde hat sich die vorliegende Erfindung die Aufgabe gestellt, eine NEM-Gußlegierung für Dentalzwecke zu entwickeln, die noch preisgünstiger als die gebräuchlichen Kobalt-Chrom-Legierungen ist. Dabei war aber zugleich zu beachten, daß die angestrebte Senkung der Materialkosten nicht durch kostensteigernden Mehraufwand erkauft werden mußte, beispielsweise bei der Werkstofferzeugung, bei der Herstellung der prothetischen Gußstücke oder bei deren anschließender Fertigbearbeitung, und daß keine Beeinträchtigung der Gebrauchseigenschaften eintrat.

Zur Lösung dieser Aufgabe wird erfindungsgemäß eine Legierung für Dentalzwecke auf Kobalt-Chrom-Basis mit folgender Zusammensetzung vorgeschlagen: (in Massen-%)

0,1 bis 1,0 % Kohlenstoff
0,05 bis 0,5 % Stickstoff
0,5 bis 3,0 % Silizium
0,3 bis 10,0 % Mangan
20 bis 35 % Chrom
2 bis 10 % Molybdän
5 bis 40 % Eisen
Rest mindestens jedoch 20 % Kobalt
einschließlich unvermeidbarer Verunreinigungen.

Bevorzugt besteht die Gußlegierung aus (in Massen-%)

0, 1 bis 0, 5 % Kohlenstoff
0, 05 bis 0, 5 % Stickstoff
0, 9 bis 2 % Silizium
1 bis 6 % Mangan
26 bis 31 % Chrom
2 bis 6 % Molybdän
20 bis 35 % Eisen
Rest mindestens jedoch 20 % Kobalt
einschließlich unvermeidbarer Verunreinigungen.

Der Gesamtgehalt an Kohlenstoff und Stickstoff soll nicht mehr als 1 % betragen.

Zur Verbesserung der Festigkeit kann die Legierung bevorzugt bis zu jeweils 0, 5 %, vorzugsweise 0, 01 bis 0, 2 %, Aluminium, Bor, Niob und Tantal einzeln oder zu mehreren enthalten.

Legierungen der beanspruchten Zusammensetzung haben eine 0, 2 %-Dehngrenze von mindestens 500 N/mm$^2$ bei einer Bruchdehnung $A_5$ nicht unter 3 %.

Bei höheren Eisengehalten im Bereich von 25 bis 40 % sollten mindestens einzelne der übrigen Elemente zur Kompensation der erhöhten Korrosionsgefahr aus ihrem oberen Gehaltsbereich ausgewählt werden.

Neben der Substitution von Kobalt durch Eisen zwecks Kostenerniedrigung werden zur Einstellung der gleichzeitig erforderlichen Werkstoffeigenschaften hinsichtlich Erzeugung, Verarbeitung und Gebrauch noch weitergehende legierungstechnische Maßnahmen getroffen. Diese bestehen in erster Linie in einer weitreichenden Variation derjenigen Elemente, die auch Bestandteil der bekannten Kobalt-Chrom-Legierungen sind. Durch diese Variationen wird eine günstige Beeinflussung des Schmelz- und Gießverhaltens sowie der Festigkeit durch die vorgeschlagenen

Zusätze an Stickstoff, Aluminium, Bor, Niob und/oder Titan bewirkt. Die Festigkeit kann auch schon durch Variation der in der Grundzusammensetzung enthaltenen Legierungselemente beeinflußt werden.

Die chemische Zusammensetzung von Beispielen der erfindungsgemäßen Legierung ist in Tafel 1 enthalten. In Tafel 2 sind Angaben über die entsprechenden mechanischen Eigenschaften und das Korrosionsverhalten der Legierungen aus Tafel 1 gemacht.

In Tafel 2 sind zum Vergleich auch repräsentative Meßergebnisse für die bekannten Co-Cr-Legierungen (A) und für eine Edelmetall-Legierung (B) mit 76 % Edelmetallanteil aufgeführt.

Für die Beispiele der erfindungsgemäßen Legierung geht aus den Ergebnissen über die 0,2%-Dehngrenze hervor, daß der Wert mit zunehmendem Gehalt an Eisen auf Kosten von dem an Kobalt abnimmt. Wie ersichtlich, ist jedoch durch die bereits erläuterten legierungstechnischen Maßnahmen gewährleistet, daß die Anforderung der ISO-Norm bezüglich der 0,2 %-Dehngrenze von mindestens 500 N/mm$^2$ auch bei den höchsten Eisengehalten nicht unterschritten wird. Im allgemeinen liegen die Werte sogar deutlich höher und erfüllen insoweit auch noch die verschärfte Anforderung von mindestens 550 N/mm$^2$ der deutschen Norm für "Kobalt-Chrom-Gußlegierungen" DIN 13 912 - März 1982.

Auch die Werte für die Bruchdehnung liegen bei den erfindungsgemäßen Beispielen erheblich über dem von den zitierten Normen einheitlich geforderten Mindestwert von 3 %. Dadurch ist sichergestellt, daß die prothetischen Gußobjekte im Bedarfsfall nachgerichtet werden können, und mitangegossene Klammern ohne Bruchgefahr zu aktivieren sind.

Der Vergleich der erfindungsgemäßen Legierungen mit der bekannten Co-Cr-Legierung A zeigt hinsichtlich der mechanischen Eigenschaften (Tafel 2), daß sie zur Hälfte dieser ebenbürtig oder sogar überlegen sind.

Die Anforderung der ISO- und DIN-Norm an die chemische Zusammensetzung, daß der Gesamtgehalt an Kobalt und Chrom mindestens 85 % betragen soll, wird nur von den Beispielen 1, 2, 10 bis 13, 17, 18 erfüllt. Daher wurden gemäß den Auflagen der ISO-Norm an fünfzehn in der chemischen Zusammensetzung repräsentativen Legierungen Korrosionsprüfungen in künstlichem Speichel nach Fusayama u.a. (siehe Journal of Dental Research, 42 (1963), Seite 1183) durchgeführt. Die hierbei erhaltenen Werte für das Durchbruchspotential, das als eine kennzeichnende Größe für die Korrosionsbeständigkeit gilt, sind Tafel 2 zu entnehmen. Danach liegt das Durchbruchspotential der erfindungsgemäßen Legierungen mit Werten zwischen + 790 und + 1050 $mV_h$ mehr oder weniger deutlich über dem Vergleichswert von + 760 $mV_h$ der bekannten Kobalt-Chrom-Legierung A.

Überraschenderweise hat sich ergeben, daß das Durchbruchspotential der erfindungsgemäßen Legierungen mit dem Eisengehalt steigt. Die so erreichten Maximalwerte von + 1000 bis + 1050 $mV_h$ entsprechen dem Vergleichswert von + 1000 $mV_h$ der angeführten Edelmetall-Legierung B.

Zusätzliche Korrosionsuntersuchungen haben noch gezeigt, daß die erfindungsgemäßen Beispiele auch absolut resistent gegenüber Spaltkorrosion und Lochfraß sind, was nicht erwartet werden konnte.

Aufgrund dieser Ergebnisse der Korrosionsprüfung ist erwiesen, daß die erfindungsgemäße Legierung unter Mundbedingungen korrosionsbeständig ist. Insoweit entsprechen auch die Ausführungsbeispiele 3 bis 9 und 14 bis 16 den Anforderungen der ISO-Norm an die chemische Zusammensetzung, obwohl ihr Gesamtgehalt an Kobalt und Chrom unter 85 % liegt.

Aufgrund der erwiesenen Korrosionsbeständigkeit der erfindungsgemäßen Legierung kann bei der gewählten Zusammensetzung auch davon ausgegangen werden, daß sie hinsichtlich der Toxizität ebenfalls als unbedenklich eingestuft werden kann, so daß sie auch in dieser Beziehung die normenmäßigen Anforderungen erfüllt.

In Ergänzung zu den dargelegten Prüfungen der Werkstoffeigenschaften an Proben, die für die Bewertung der Gebrauchseigenschaften Bedeutung haben, sind an den Beispielen der Tafel 1 auch umfassende Eignungsprüfungen im Dentallabor durchgeführt worden. Aufgrunddessen läßt sich die erfindungsgemäß vorgeschlagene Legierung in jeder Hinsicht ebenso problemlos schmelzen, vergießen und bearbeiten, wie die bekannten Co-Cr-Legierungen.

Die vorgelegten Prüfungsergebnisse weisen in ihrer Gesamtheit aus, daß die vorgeschlagene Legierung einen vollwertigen Ersatz für die bekannten Co-Cr-Legierungen darstellt und wegen ihres geringeren Preises einen Beitrag zur Kostendämpfung im Gesundheitswesen leisten kann.

0195351

Tafel 1: Chemische Zusammensetzung

| Schmelze | Masse-% | | | | | | | | | Son-stige |
|---|---|---|---|---|---|---|---|---|---|---|
| | C | Si | Mn | Cr | Fe | Mo | Al | B | N | |
| 1 | 0,55 | 0,62 | 0,31 | 27,70 | 5,10 | 5,20 | 0,22 | - | 0,26 | - |
| 2 | 0,60 | 1,25 | 1,09 | 30,20 | 10,22 | 2,49 | 0,15 | - | 0,14 | - |
| 3 | 0,59 | 1,18 | 1,12 | 30,10 | 19,25 | 2,42 | 0,17 | - | 0,15 | - |
| 4 | 0,59 | 0,84 | 0,87 | 30,00 | 28,48 | 2,05 | 0,017 | - | 0,10 | - |
| 5 | 0,29 | 0,90 | 0,86 | 30,35 | 28,91 | 2,05 | 0,017 | - | 0,078 | - |
| 6 | 0,30 | 0,86 | 0,73 | 28,15 | 29,50 | 2,37 | n.b. | - | 0,21 | - |
| 7 | 0,28 | 1,01 | 0,81 | 28,35 | 29,00 | 5,16 | n.b. | - | 0,32 | - |
| 8 | 0,85 | 1,53 | 1,13 | 29,44 | 28,90 | 2,36 | 0,39 | - | 0,14 | - |
| 9 | 0,35 | 1,22 | 1,10 | 28,15 | 39,60 | 4,95 | 0,05 | 0,020 | 0,37 | - |
| 10 | 0,14 | 0,70 | 0,39 | 27,85 | 5,15 | 5,20 | 0,35 | n.b. | 0,27 | - |
| 11 | 0,13 | 0,64 | 0,35 | 27,42 | 5,07 | 5,07 | n.b. | 0,024 | 0,24 | 0,26 Nb |
| 12 | 0,13 | 0,71 | 0,35 | 27,84 | 5,12 | 5,01 | n.b. | 0,024 | 0,23 | 0,11 Ti |
| 13 | 0,12 | 0,95 | 2,76 | 27,79 | 2,35 | 5,14 | n.b. | 0.021 | 0,22 | - |
| 14 | 0,11 | 1,16 | 0,30 | 30,10 | 20,00 | 2,47 | 0,02 | - | 0,22 | - |
| 15 | 0,14 | 1,13 | 1,03 | 20,49 | 22,23 | 2,55 | 0,03 | - | 0,11 | - |
| 16 | 0,13 | 1,18 | 0,92 | 20,39 | 33,16 | 2,60 | 0,02 | - | 0,31 | - |
| 17 | 0,13 | 1,96 | 3,23 | 27,68 | 4,57 | 5,14 | - | - | 0,23 | - |
| 18 | 0,14 | 1,38 | 5,06 | 27,63 | 4,91 | 5,54 | - | - | 0,23 | - |

Rest Cobalt einschließlich unvermeidbarer Verunreinigungen

Tafel 2: Eigenschaften                    0195351

| Schmelze | 0,2 %-Dehngrenze N/mm$^2$ | Bruchdehnung A$_5$ % | Durchbruchspotential mV$_h$ [1] |
|:---:|:---:|:---:|:---:|
| A | 635 | 9,0 | + 760 |
| B | - | - | + 1000 |
| 1 | 680 | 4,6 | - |
| 2 | 620 | 4,5 | + 850 |
| 3 | 600 | 6,6 | + 930 |
| 4 | 560 | 5,4 | + 1000 |
| 5 | 550 | 6,4 | + 1050 |
| 6 | 560 | 9,0 | + 1000 |
| 7 | 570 | 9,2 | + 930 |
| 8 | 615 | 3,5 | + 980 |
| 9 | 650 | 3,6 | + 1040 |
| 10 | 625 | 8,1 | + 830 |
| 11 | 655 | 13,1 | - |
| 12 | 630 | 9,1 | - |
| 13 | 655 | 13,3 | + 790 |
| 14 | 535 | 12,5 | + 980 |
| 15 | 510 | 13,3 | + 910 |
| 16 | 570 | 13,6 | + 980 |
| 17 | 670 | 4,7 | + 830 |
| 18 | 665 | 8,0 | + 840 |

A = gebräuchliche Co-Cr-Legierung B = " Au-Pt-Pd-Legierung 1) gemessen bei 37°C an Luft in künstlichem Speichel nach Fusayama

- 10 -                    0195351

Patentansprüche

1. Verwendung einer Kobalt-Chrom-Gußlegierung mit (in Massen-%)

0,1 bis 1,0 % Kohlenstoff
0,05 bis 0,5 % Stickstoff
0,5 bis 3,0 % Silizium
0,3 bis 10,0 % Mangan
20 bis 35 % Chrom
2 bis 10 % Molybdän
5 bis 40 % Eisen
Rest mindestens jedoch 20 % Kobalt
einschließlich unvermeidbarer Verunreinigungen

als Werkstoff für gegossene Gerüste und Platten für herausnehmbare Dentalprothesen.

2. Verwendung einer Kobalt-Chrom-Gußlegierung nach Anspruch 1 mit (in Massen-%)

0,1 bis 0,5 % Kohlenstoff
0,05 bis 0,5 % Stickstoff
0,9 bis 2 % Silizium
1 bis 6 % Mangan
26 bis 31 % Chrom
2 bis 6 % Molybdän
20 bis 35 % Eisen
Rest mindestens jedoch 20 % Kobalt
einschließlich unvermeidbarer Verunreinigungen

für den Zweck nach Anspruch 1.

3. Verwendung einer Legierung der Zusammensetzung nach einem der Ansprüche 1 oder 2 mit einem Gesamtgehalt an Kohlenstoff und Stickstoff von höchstens 1 % für den Zweck nach Anspruch 1.

4. Verwendung einer Legierung der Zusammensetzung nach Anspruch 1 oder 2, die jedoch zur Verbesserung der Festigkeit zusätzlich bis zu jeweils 0,5 % Aluminium, Bor, Niob, Titan einzeln oder zu mehreren enthält, für den Zweck nach Anspruch 1.

5. Verwendung einer Legierung der Zusammensetzung nach einem der Ansprüche 1 oder 2 mit 4, die jedoch zur Verbesserung der Festigkeit zusätzlich jeweils 0,01 bis 0,2 % Aluminium, Bor, Niob, Titan einzeln oder zu mehreren enthält, für den Zweck nach Anspruch 1.

6. Verwendung einer Legierung der Zusammensetzung nach einem der Ansprüche 1, 2, 4 oder 5, die eine 0,2 %-Dehngrenze von mindestens 500 $N/mm^2$ bei einer Bruchdehnung $A_5$ nicht unter 3 % besitzt, wobei bei höheren Eisengehalten von 25 bis 40 % mindestens einzelne der übrigen Elemente aus ihrem oberen Gehaltsbereich ausgewählt werden, für den Zweck nach Anspruch 1.